# EUROPEAN PATENT APPLICATION

(11) **EP 2 050 433 A1**
(43) Date of publication of application: **22.04.2009**
(21) Application number: 07118589.6
(22) Date of filing: 16.10.2007
(51) Int. Cl.: A61K 8/11, A61K 8/37, A61K 8/81, A61Q 13/00, A61Q 19/00

(54) **Cosmetic composition comprising solid entrapping particles for sustained release of volatile materials**

(71) Applicant: The Procter and Gamble Company, Cincinnati, Ohio 45202 (US)
(72) Inventor: D'acchioli, Vincenzo, 65125 Pescara (IT); Dini, Liana Amneris, N98 Vasto (CH) (IT); Guarracino, Mario, 64029 Silvi Marina (Teramo) (IT); Turacchio, Sabrina, 65025 Pescara (IT)
(74) Representative: Wilding, Richard Alan

(57) **Abstract**

The invention relates to a cosmetic composition comprising (a) solid entrapping particles having an average particle size from 5 to 200 micrometers and comprising (i) a copolymer obtainable by copolymerization of monomers of ethylene with at least one class of comonomers comprising at least one heteroatom, (ii) a plasticizer comprising at least one heteroatom, and (iii) a volatile material; and (b) a cosmetically acceptable carrier.

## Description

### FIELD OF THE INVENTION

The present invention relates to a cosmetic composition comprising a cosmetically acceptable carrier and solid entrapping particles having an average particle size from 5 to 200 micrometers. The cosmetic composition provides a sustained, uniform release of volatile materials without compromising the skin feel and without inducing any skin irritation.

### BACKGROUND OF THE INVENTION

Cosmetic compositions may comprise volatile materials, such as perfumes, to impart a pleasant scent while the consumer is using the compositions. Due to their volatility, these materials, or at least the more highly volatile portions of them, tend to evaporate quickly after application of the cosmetic composition onto the skin and consequently the pleasant effects of the perfume dissipate within a short period of time. In addition, once the container comprising the cosmetic composition has been opened, these volatile materials tend to evaporate and cosmetic compositions tend to lose their scent over the time upon storage.

In order to limit the loss of volatile materials over time and/or to provide a sustained release of volatile materials, technologies based on the encapsulating or the entrapping of volatile materials into polymeric compositions have been developed. See, for example EP 1 624 013, EP 1 604 690, WO 01/93813, WO 01/93814 and WO 01/93815.

Nevertheless, there is still a need to provide a cosmetic composition, particularly a leave-on topical composition, exhibiting an improved sustained, uniform release of volatile materials over time. There is also a need for providing a cosmetic composition which does not compromise the skin feel and does not induce skin irritation.

### SUMMARY OF THE INVENTION

The present invention relates to a cosmetic composition comprising:
(a) solid entrapping particles, these particles comprising
   (i) a copolymer obtainable by copolymerization between monomers of ethylene and at least one class of co-monomers comprising at least one heteroatom;
   (ii) a plasticizer comprising at least one heteroatom;
   (iii) a volatile material;
(b) a cosmetically acceptable carrier;
wherein the solid entrapping particles have an average particle size from 5 to 200 micrometers.

### Definitions

As used herein, the term "sustained release" means that the volatile material is released over a long period of time. Particularly, the term "sustained" release means that the volatile material is still noticeable by the consumer more than four hours after the application of the cosmetic composition onto the skin.

The term "uniform release" means the scent perceived by the consumer is substantially identical over a long period of time. Particularly, the term "uniform release" means that the scent perceived by the consumer more than four hours after the application of the personal care composition onto the skin is substantially identical to the scent the consumer perceived immediately after the application of this composition onto the skin.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

### DETAILED DESCRIPTION OF THE INVENTION

The cosmetic composition according to the present invention comprises solid entrapping particles. These solid entrapping particles are obtainable from a polymeric composition comprising a copolymer obtainable by copolymerization between monomers of ethylene and at least one class of co-monomer comprising at least one heteroatom, a plasticizer comprising at least one heteroatom and a volatile material.

The inventors have surprisingly found that a cosmetic composition, particularly a leave-on topical cosmetic composition, providing a sustained, uniform release of volatile materials without compromising the skin feel and/or without inducing any skin irritation can be achieved when the volatile materials are comprised within solid entrapping particles. In particular, such benefits are achieved when these solid entrapping particles have an average particle size from 5 to 200 micrometers. Without wishing to be bound by theory, it is believed that the composition of the solid entrapping particles in combination with their average particle size in the claimed range prevent the volatile materials and/or the solid particles themselves to be absorbed by the skin to a level that would induce skin irritation but without preventing an appropriate diffusivity of the volatile materials through the solid particles to achieve a sustained, uniform release over time. In addition, particles having an average particle size in the claimed range demonstrate beneficial perfume release rates on account of their advantageous surface area/volume ratio.

### Shape of the solid entrapping particles

The solid entrapping particles can be of different shapes depending on the process used and the cosmetic composition into which they are incorporated. For example, when dispersed in an aqueous solution, solid particles generally have a substantially spherical shape. When dispersed in an emulsion, solid particles have a more irregular shape, e.g. the shape of an irregular star. Without wishing to be bound by theory, it is believed that the shape of the solid entrapping particles is dependent, at least partially, on the difference of surface energy between the cosmetically acceptable carrier and the solid entrapping particles. When the difference is maximal, the solid entrapping particles are spherical. As the difference of surface energy diminishes, the solid entrapping particles become more irregular in shape. Whatever the shape of the solid particles, the inventors have found that a sustained, uniform release of volatile materials without compromising the skin feel and without any skin irritation can be achieved when solid entrapping particles have an average particle size from 5 to 200 micrometers, preferably from 30 to 100 micrometers, more preferably from 30 to 50 micrometers. The average particle size can be determined by using any appropriate instrument known by the skilled person. For example, the average particle size may be measured with Lasentec D600L instrument, supplied by Mettler Toledo, using a technique known as Focused Beam Reflectance Measurement (FBRM) which provides continuous in-process and real-time measurement of the rate and degree of change of the particle size and particle count. In this technique, a focused laser beam rotates at high speed and propagates into the particle suspension to be monitored. When the focused laser beam crosses a particle in front of the probe, a signal is backscattered into the probe. The length of the scanned particle (or size) is determined by the electronics of the system and transferred into a diameter length distribution histogram. The diameter length distribution provides particle count and particle size information from which an average particle size may be determined.

### Copolymer

The solid entrapping particles according of the invention comprise a copolymer obtainable by copolymerization between monomers of ethylene and at least one class of co-monomers comprising at least one heteroatom. The copolymer comprises, therefore, ethylene monomers and co-monomers comprising at least one heteroatom.

The term "co-monomer comprising at least one heteroatom" includes monomers comprising at least a polar C-X linkage with X being neither a carbon atom nor a hydrogen atom. X can be a nitrogen, sulphur, fluor, chlorine or oxygen atom. Preferably, the co-monomer comprises a carbonyl group, more preferably the co-monomer comprises an ester group selected from a vinyl ester, an acrylic ester, a methacrylic ester and mixture thereof, still more preferably the co-monomer is a vinyl ester. The most preferred co-monomer is a vinyl acetate ester and the copolymer is, therefore, an ethylene-vinyl acetate copolymer (or EVA copolymer). Without wishing to be bound by theory, it is believed that EVA copolymers are the most effective copolymer in increasing the sustained release of volatile materials.

The co-monomer preferably represents from 10% to 90%, more preferably from 14% to 80%, still more preferably from 18% to 70% by weight of the total copolymer.

The copolymer may further comprise other co-monomers not being a co-monomer comprising at least one heteroatom. For example, the copolymer may further comprise crosslinking co-monomers.

These copolymers have a low melting point. As used herein, the term "low melting point" means a melting point below 80°C, preferably from 45°C to 80°C and more preferably from 45°C to 55°C. The melting point can be measured by conventional methods. Copolymers having a low melting-point are preferred so that the polymeric composition can be formulated at a low application temperature, i.e. at a temperature below 100°C, and then these polymeric compositions can be incorporated into the cosmetic composition also at a low application temperature. The use of a low application temperature is desirable to prevent the evaporation of a significant amount of volatile materials and also to prevent the degradation and/or the inactivation of cosmetic active components when incorporating the polymeric composition into the cosmetic composition.

Copolymer raw materials are commercially available. For example, suitable ethylene-vinyl acetate copolymer raw materials are sold under the trade names: Elvax^{™} by Dupont, Evathane^{™} by Atofina, Escorene^{™} by Exxon, Levamelt^{™} and Levamelt^{™} by Bayer. Particularly preferred are Elvax^{™} 250 having a melting point of 70°C and Elvax^{™} 40W having a melting point of 47°C. A suitable ethylene-acrylic esters copolymer raw material is sold under the trade name Lotryl^{™} by Atofina.

### Plasticizer comprising at least one heteroatom

The solid entrapping particles comprise a plasticizer comprising at least one heteroatom. This plasticizer comprising at least one heteroatom is compatible with the copolymer obtainable by copolymerization between monomers of ethylene and at least one class of co-monomers comprising at least one heteroatom.

As used herein, the term "compatible" means that the solubility of the copolymer matches the solubility of the plasticizer at ambient temperature (i.e. at 25°). When a copolymer and a compatible plasticizer are mixed, an homogeneous mixture is obtained and the compatible plasticizer exhibits a plasticizing effect as it lowers the glass transition temperature of the polymer. The compatibility of the copolymer and the plasticizer may be determined by using any conventional method known by the skilled person, e.g. water/octanol partition coefficient.

The term "plasticizer comprising at least one heteroatom" includes plasticizers comprising at least a polar C-X linkage wherein X is neither a hydrogen atom nor a carbon atom. X can be nitrogen, sulphur, fluor, chlorine or oxygen atom. Preferably, the plasticizer comprises a carbonyl group, more preferably an ester group. When the plasticizer comprises an ester group, the plasticizer is preferably selected from an adipic acid ester, a benzoic acid ester, a citric acid ester, a phosphoric acid ester, a phthalic acid ester, a rosin ester, a sebacic acid ester, a sucrose ester, a tartaric acid ester or mixture thereof. More preferably, the plasticizer is selected from an adipic acid ester, a citric acid ester, a rosin ester, a sebacic acid ester or mixture thereof. Still more preferably, the plasticizer is selected from citric esters and/or sebacic esters as both citric esters and/or sebacic esters exhibit a satisfactory compatibility with the copolymer according to the invention and are safe for skin application.

Plasticizer raw materials are commercially available. For example, suitable esters of hydrogenated rosin are sold under the trade name Foralyn 5020-F™ by Eastman. Suitable esters of citric acid are sold under the trade name Citrofol BII™ (acetyl tributyl citrate) by Jungbunzlauer; Citrofol AH II™ (acetyl 2-ethylhexyl citrate) by Jungbunzlauer, and; Morflex MSC™ (monosteryl citrate) by Morflex. Suitable esters of sebacic acid are sold under the trade name Morflex DBS™ (dibutyl sebacate) by Morflex.

### Volatile materials

The solid entrapping particles comprise at least one volatile material.

The volatile materials can be selected from flavors, deodorants, insecticides, pheromones, aromas, repelling agents, perfumes or a mixture thereof.

Preferably, the volatile material is a perfume. As used herein, the term "perfume" means any odiferous material comprising one or more volatile components, i.e. components having a vapor pressure less than the atmospheric pressure at room temperature. Depending on their volatility, these components tend to evaporate more or less quickly. The higher the volatility is, the quicker the evaporation. Components evaporating the most rapidly are usually classified as top notes and components evaporating the least rapidly as base notes, intermediary components being classified as middle notes.

More preferably, perfume comprises more than one component having a vapor pressure less than the atmospheric pressure at room temperature. When said perfume comprises more that one of these components, these components usually have different volatility. When these components are merely incorporated into a cosmetic composition, these components tend to evaporate at different speed depending on their volatility upon use. Due to this differential evaporation, the consumer perceives different scents over time. In contrast, when a cosmetic composition comprises a perfume comprised within the solid entrapping particles according to the invention, the release of the perfume would be uniform over time. Without wishing to be bound by theory, it is believed that the release of these volatile components over the time is not merely driven by their volatility. In contrast, it is believed that their release is dependent to several factors - including their volatility, their molecular weight, their chemical interactions with the other components of the solid entrapping particles and the total proportion of volatile materials in the solid entrapping particles in combination with the specific average particle size of the solid entrapping particles - driving therefore a sustained, uniform release over the time.
Perfume can be in a liquid or solid form at room temperature.
Perfume can be obtained from a natural source, a synthetic source or a mixture thereof. Preferably, the perfume comprises at least one component being selected from an aldehyde, a ketone, an ester, an alcohol or a terpene.

These components may be selected depending on the scent desired. These components are well-known by the skilled person. For illustration, perfume materials are described more fully in S. Artander, Perfume Flavors and chemicals, Vols I and II, Montclair, N.J. and the Merck Index, 8th Edition, Merck & Co., Rahway N.J., which are incorporated herewith by reference.

Preferably, the solid entrapping particles comprise from 5% to 75%, more preferably from 10% to 50%, copolymer obtainable by copolymerization between monomers of ethylene and at least one class of co-monomers comprising at least one heteroatom, from 10% to 50%, more preferably from 15 to 40%, plasticizer comprising at least one heteroatom and from 10 to 85%, more preferably from 20 to 85%, still more preferably from 30% to 85%, and most preferably from 40% to 85%, volatile material by weight of particle. The solid entrapping particles allow the incorporation of a high proportion of volatile materials.

### Cosmetically acceptable carrier

The cosmetic composition according to the present invention comprises a cosmetically acceptable carrier. This cosmetically acceptable carrier can be any cosmetically acceptable carrier conventionally used in cosmetics, particularly an aqueous solution or an emulsion. If it is an emulsion, then the cosmetic composition may be a water-in-oil emulsion, an oil-in-water emulsion or a multiple emulsion.

### Form of the composition

The cosmetic composition is preferably a leave-on, topical, cosmetic composition. By the term "leave-on", it is meant a cosmetic composition that is applied onto the skin and which is not rinsed-off after application. Preferably, a leave-on, topical, cosmetic composition is a composition that persists onto the skin for at least four hours.

### Proportions of solid entrapping particles

The cosmetic composition comprises preferably from 0.01 % to 30%, more preferably from 0.2% to 10%, still more preferably from 0.5% to 6% solid entrapping particles by weight of the composition.

The cosmetic composition according to the invention may comprise optional ingredients. In particular, this cosmetic composition may comprise ingredients being commonly used in cosmetic compositions.

The cosmetic composition may further comprise at least one humectant. Preferably, the humectant comprises polyhydric alcohols. More preferably, the humectant comprises glycerine, propylene glycol, dipropylene glycol, polypropylene glycol, polyethylene glycol, sorbitol, hydroxypropyl sorbitol, hexylene glycol, 1,3-butylene glycol, 1,2,6-hexanetriol, ethoxylated glycerin, propoxylated glycerin or mixtures thereof. Still more preferably, the humectant comprises glycerine.

The cosmetic composition may further comprise at least one thickener. Thickeners which may be employed comprise C₁₂-C₂₄ fatty acids, C₁₀-C₃₀ fatty alcohols, N-fatty glutamic acid dialylamides, carboxy methyl cellulose or derivatives thereof, polysaccharide gum, starch or derivatives thereof, particulate thickeners, carboxyvinyl polymers, sodium acrylate copolymers and hydrophobically modified sodium acrylate copolymers, polyacrylamide copolymers, polyacrylates, acrylamide/ammonium acrylate copolymer, ammonium acryloyldimethyl taurate and ammonium acryloyldimethyl taurate crosspolymers and copolymers and hydrophobically modified ammonium acryloyldimethyl taurate copolymers, cationionically charged polymers, or mixtures thereof. Preferably, the thickener comprises Carbomer polymers.

The cosmetic composition may further comprise a hydrophilic vitamin component. Preferably, the hydrophilic vitamin component comprises vitamin B₃, salts or esters thereof; provitamin B₅, salts or esters thereof; vitamin C, salts or esters thereof; hydrophilic derivatives of vitamin E, or mixtures thereof.

The cosmetic composition may further comprise a hydrophobic vitamin component. The hydrophobic vitamin component may comprise vitamin E or hydrophobic derivatives thereof, vitamin D or derivatives thereof, or mixtures thereof.

The cosmetic composition may further comprise at least one sunscreen active, i.e. an hydrophilic organic sunscreen active, an hydrophobic organic sunscreen active, an inorganic sunscreen or mixtures thereof. Suitable examples of sunscreens may be found in the CTFA International Cosmetic Ingredient Dictionary and Handbook, 7th edition volume 2, pp.1672, edited by Wenning and Mc Ewen (The Cosmetic, Toiletry, and Fragrance Association, Inc., Washibgton, D.C. 1997). Preferably, the hydrophobic organic sunscreen active comprises alkyl β,β-diphenylacrylate derivatives, α-cyano β,β-diphenylacrylate derivatives, anthranilate derivatives, benzophenone derivatives, cinnamic derivatives, camphor derivatives, dibenzoylmethane derivatives, p-aminobenzoic derivatives, salicylic derivatives, triazine derivatives, or mixtures thereof. Preferably, the hydrophilic organic sunscreen active is 2-phenylbenzimidaole-5-sulfonic acid (PBSA). Preferably, the inorganic sunscreen active comprises titanium dioxide, zinc oxide, or mixtures thereof.

The cosmetic composition may further comprise at least one tanning active. Preferably, said tanning active is selected from dihydroxyacetone (DHA), salts, derivatives or tautomers thereof, or mixtures thereof.

The cosmetic composition may further comprise at least one sugar amine. Said sugar amine can be synthetic or natural in origin and can be used as pure compounds or mixtures of compounds. Preferably, sugar amine comprises glucosamine, N-acetyl glucosamine, mannosamine, N-acetyl mannosamine, galactosamine, N-acetyl galactosamine, isomers thereof, salts thereof, or mixtures thereof.

The cosmetic composition may further comprise at least one hexaminidine compound, salts or derivatives thereof, or mixtures thereof. Preferably, hexaminidine compound comprises compounds corresponding to the following chemical structure: 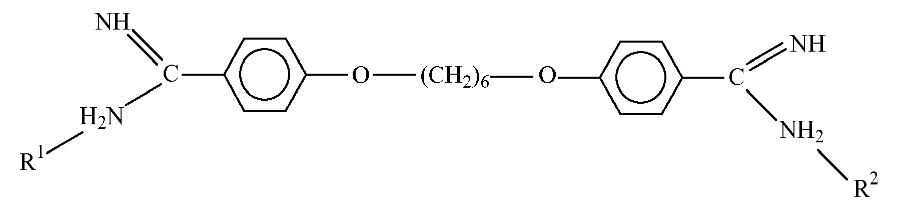 wherein R¹ and R² are organic acids (e.g., sulfonic acids, etc.).

When the cosmetic composition is in the form of an oil-in-water emulsion, this composition may further comprise at least one hydrophilic surfactant. Generally, hydrophilic surfactants help disperse and suspend the discontinuous phase within the continuous phase. Preferably, hydrophilic surfactants are selected from nonionic surfactants such as those known in the art. Other suitable surfactants useful herein comprise a wide variety of cationic, anionic, zwitterionic, and amphoteric surfactants such as are known in the art. See, e.g., McCutcheon's, Detergents and Emulsifiers, North American Edition (1986), published by Allured Publishing Corporation. The hydrophilic surfactants useful herein can contain a single surfactant, or any combination of suitable surfactants. The surfactant(s) chosen will depend upon the pH of the composition and the other components present.

The cosmetic composition may further comprise at least one oily component such as a natural or synthetic oils selected from mineral, vegetable and animal oils, fats and waxes, fatty acid esters, fatty alcohols, fatty acids, or mixtures thereof. Preferably, the cosmetic composition may comprise saturated and unsaturated fatty alcohols such as behenyl alcohol, cetyl alcohol and stearyl alcohol.

The cosmetic composition may further comprise at least one emollient. Preferably, this composition comprises a branched chain hydrocarbons having a weight average molecular weight of from 100 to 15,000, more preferably from 100 to 1000, still more preferably a branched chain hydrocarbonsselected from isododecane, isohexadecane, isoeicosane, isooctahexacontane, isohexapentacontahectane, isopentacontaoctactane, petrolanum, isopropyl palmitate, isopropyl isostearate, or mixture thereof.

The cosmetic composition may further comprise at least one particulate material. Particulate materials may comprise colored and uncolored pigments, interference pigments, inorganic powders, composition powders, optical brightener particles, or mixture thereof. These particulate materials do not comprise inorganic sunscreen active. Preferably, particulate materials comprise free-flowing, solid (i.e. not hollow) materials that are insoluble in both water and oil, with a median particle size of from 0,1 µm to 75 µm, more preferably from 0,2 µm to 30µm, and with a refractive index of from 1.3 to 1.7, said materials being dispersed in the composition. Suitable particulate materials are organic or organosilicone or inorganic.

The cosmetic composition may further comprise a structuring agent. Preferably, structuring agents are selected from stearic acid, palmitic acid, stearyl alcohol, cetyl alcohol, behenyl alcohol, steareth-2, steareth-21, or mixtures thereof.

The cosmetic composition may also comprise, in addition to the volatile materials comprised within the solid entrapping particles, further volatile materials including essential oils, fragrances or mixtures thereof.

A variety of additional optional ingredients may be incorporated into the cosmetic composition according to the invention. Non-limiting examples of these additional ingredients include additional skin care actives such as bisabolol, dialkanoyl hydroxyproline compounds, farnesol, flavonoids, guanidine, N-acyl amino acid compounds, peptides, phytantriol, phytosterols, salicylic compounds, urea as well as compounds such as anti-acne compounds, antioxidants compounds, skin soothing and healing agents, anti-wrinkle/anti-atrophy actives, conditioning agents, anti-inflammatory agents, skin lightening agents, antimicrobial/antibacterial/antifungal actives, chelators and sequestrants.

### Method of manufacture

The polymeric composition may be prepared with any conventional method.

Preferably, the polymeric composition is prepared according to the conventional hot melt blending method. A polymeric composition comprising a copolymer obtainable by copolymerization between monomers of ethylene and at least one class of co-monomers comprising at least one heteroatom, a plasticizer comprising at least one heteroatom and a volatile material is prepared prior dispersion into the cosmetic composition. The copolymer and the plasticizer are mixed and melted at a temperature above their melting point till the obtaining of a homogeneous mixture. When the mixture is homogeneous, the temperature is cooled down and then the volatile material is added to the polymeric composition under stirring conditions till the obtaining of a homogenous mixture and the polymeric composition comprising volatile material is cooled down at room temperature.

Before dispersing the solid entrapping particles into the cosmetic composition, the polymeric composition comprising volatile material is melted at 60-70°C. The melted polymeric composition is then dispersed under stirring conditions into the cosmetic composition. The stirring conditions determine the average particle size of the solid entrapping particles of polymeric composition.

### Example 1 - Preparation of the polymeric composition

A polymeric composition is prepared comprising 30% of Elvax 250™ (EVA copolymer), 20% of Foralyn 5020-F™ (plasticizer) and 50% perfume oil. Elvax 250™ and Foralyn 5020-F™ are melted and mixed at a temperature above their melting point, i.e. at 60-70°C till an homogenous mixture is obtained. Then, this mixture is cooled down at approximately 50°C before adding the volatile material under stirring conditions so as to obtain a homogenous polymeric composition.

Then, the resulting polymeric composition is cooled down at room temperature.

### Example 2 - Body lotion

The polymeric composition of example 1 is melted at 60-70°C before being dispersed into the aqueous solution at 60-70°C and under agitation at 3500-4000 rpm. Once the polymeric composition has been homogenously dispersed, the aqueous solution is cooled down to 45-50 °C, the remaining ingredients are added under stirring conditions till a homogeneous composition is obtained. The aqueous solution is used as a leave-on body lotion.

| Ingredients | Proportions by weight of the total composition |
|---|---|
| Water | Qsp |
| Polymeric composition (50% perfume) according to example 1 | 2.0% |
| Preservatives | 0.5% |
| Glycerin | 2.0% |
| Propylene glycol | 0.15% |
| Glyceryl stearate | 0.5% |
| Dimethicone | 2.0% |
| Mineral oil | 2.0% |

### Example 3 - Body lotion

The polymeric composition of example 1 is melted at 60-70°C before being dispersed into the aqueous solution at 60-70°C and under agitation at 3500-4000 rpm. Once the polymeric composition has been homogenously dispersed, the aqueous solution is cooled down to 45-50 °C, the remaining ingredients are added under stirring conditions till a homogeneous composition is obtained. The aqueous solution is used as a leave-on body lotion.

| Ingredients | Proportions by weight of the total composition |
|---|---|
| Water | Qsp |
| Polymeric composition (50% perfume) according to example 1 | 6.0% |
| Methyl paraben | 0.12% |
| Imidazolidinyl urea | 0.2% |
| Allantoin | 0.2% |
| Disodium EDTA | 0.1% |
| Citric acid anhydrous | 0.01 % |
| Glycerin | 2.58% |
| Panthenol | 2.0% |
| Butylene glycol | 1.0% |
| Phenoxyethanol | 0.25% |
| Tocopheryl acetate | 2.0% |
| PEG-40 hydrogenated castor oil 90% | 0.5% |
| Bisabolol | 0.2% |
| Dimethicone & dimethiconol | 3.0% |

After application onto the skin of the body lotion of example 2 or example 3, the perfume is still noticeable by the user after four hours.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A cosmetic composition comprising
(a) solid entrapping particles, said particles comprising
(i) a copolymer obtainable by copolymerization of monomers of ethylene with at least one class of co-monomers comprising at least one heteroatom;
(ii) a plasticizer comprising at least one heteroatom;
(iii) a volatile material;
(b) a cosmetically acceptable carrier;
wherein the solid entrapping particles have an average particle size from 5 to 200 micrometers.

2. A composition according to claim 1 wherein said average particle size is from 30 to 100 micrometers, preferably from 30 to 50 micrometers.

3. A composition according to claim 1 or 2 wherein said composition is a leave-on topical composition.

4. A composition according to any preceding claim wherein said composition comprises from 0.01% to 30%, preferably from 0.2% to 10%, more preferably from 0.5% to 6% solid entrapping particles by weight of the composition.

5. A composition according to any preceding claim wherein said copolymer has a melting point from 45°C to 80°C, preferably from 45°C to 55°C.

6. A composition according to any preceding claim wherein said co-monomer comprises a carbonyl group, preferably said co-monomer comprises an ester group, more preferably said co-monomer is selected from a vinyl ester, an acrylic ester, a methacrylic ester and mixture thereof, still more preferably said co-monomer is a vinyl acetate ester.

7. A composition according to any of the preceding claims wherein said plasticizer comprises a carbonyl group, preferably an ester group and more preferably the plasticizer is selected from adipic acid esters, benzoic acid esters, citric acid esters, phosphoric acid esters, phthalic acid esters, rosin esters, sebacic acid esters, sucrose esters, tartaric acid esters or mixture thereof, still more preferably the plasticizer is selected from adipic acid esters, citric acid esters, rosin esters, sebacic acid esters or mixtures thereof.

8. A composition according to any of the preceding claims wherein the volatile material is a perfume, preferably the volatile material is selected from an aldehyde, a ketone, an alcohol, a terpene, an ester or mixture thereof.

9. A composition according to any of the preceding claims wherein the solid entrapping particles comprise, by weight of particle, from 5% to 75% copolymer, from 10% to 50% plasticizer and from 10 to 85% volatile material.
